# FASCICULE DE BREVET EUROPEEN

(11) **EP 4 405 665 B1**
(45) Date de publication et mention de la délivrance du brevet: **22.07.2026**
(21) Numéro de dépôt: 22777239.9
(22) Date de dépôt: 19.09.2022
(51) Int. Cl.: G01N 21/64, G01N 33/58, G01N 33/50, B82Y 5/00, B82Y 15/00

(54) **PROCÉDÉ D'ÉTUDE DU COMPORTEMENT D'UN ÉCHANTILLON CELLULAIRE**
VERFAHREN ZUR UNTERSUCHUNG DES VERHALTENS EINER ZELLPROBE
METHOD FOR STUDYING THE BEHAVIOUR OF A CELL SAMPLE

(30) Priorité: 23.09.2021 FR 2110013
(43) Date de publication de la demande: 31.07.2024
(73) Titulaire: Ecole Polytechnique, 91120 Palaiseau (FR); Centre National de la Recherche Scientifique, 75016 Paris (FR); Institut National de la Santé et de la Recherche Médicale, 75013 Paris (FR); Université Paris-Est Créteil Val de Marne, 94000 Créteil (FR)
(72) Inventeur: KIM, Jongwook, 75014 PARIS (FR); GACOIN, Thierry, 91440 Bures sur Yvette (FR); FILOCHE, Marcel, 92340 Bourg la Reine (FR); LOUIS, Bruno, 94000 CRETEIL (FR); BEQUIGNON, Emilie, 94220 Charenton le Pont (FR)
(74) Mandataire: Ipsilon
(86) Numéro de dépôt international: PCT/EP2022/075972
(87) Numéro de publication internationale: WO 2023/046634

(56) Documents cités:
- US-A1- 2010 028 543
- DASGUPTA S ET AL: "Nano- and microparticles at fluid and biological interfaces", JOURNAL OF PHYSICS: CONDENSED MATTER, INSTITUTE OF PHYSICS PUBLISHING, BRISTOL, GB, vol. 29, no. 37, 11 August 2017 (2017-08-11), pages 373003, XP020319568, ISSN: 0953-8984, [retrieved on 20170811], DOI: 10.1088/1361-648X/AA7933
- RYAN T. SHAFRANEK ET AL: "Stimuli-responsive materials in additive manufacturing", PROGRESS IN POLYMER SCIENCE, vol. 93, 1 June 2019 (2019-06-01), GB, pages 36 - 67, XP055658896, ISSN: 0079-6700, DOI: 10.1016/j.progpolymsci.2019.03.002

## Description

La présente invention porte sur un procédé d'étude du comportement d'un échantillon cellulaire contenues dans un milieu fluide. L'invention concerne également le dispositif d'étude correspondant.

### Domaine technique

Actuellement, l'étude du comportement d'un échantillon cellulaire, notamment sa viabilité et/ou ses fonctionnalités, nécessite le plus souvent l'utilisation d'outils de microscopie à haute résolution, optique ou électronique, associés à l'analyse d'images complexes et/ou à des analyses génétiques. De telles procédures sont lentes et coûteuses, ce qui les rend peu adaptées à des études cliniques, notamment dans le cas d'évaluation *in vitro* de cellules biologiques prélevées dans une pratique de soin courant.

A titre d'exemple, la clairance mucociliaire est l'un des principaux moyens de défense par lesquels les poumons évacuent en permanence des particules inhalées par les voies respiratoires. Les particules indésirables sont piégées par le mucus recouvrant l'épithélium des voies respiratoires. Les aérocontaminants et le mucus sont ensuite entraînés ensemble le long des voies respiratoires par le battement synchrone de cils présents sur l'épithélium des voies respiratoires, jusqu'à ce qu'ils soient expulsés dans l'œsophage. L'absence ou l'altération de cette clairance peut entraîner des troubles obstructifs chroniques par accumulation de mucus, troubles que l'on observe non seulement dans des maladies rares congénitales comme la mucoviscidose ou les dyskinésies ciliaires primitives (DCP), mais aussi dans des maladies beaucoup plus courantes comme les rhinites chroniques, l'asthme et la broncho-pneumopathie chronique obstructive (BPCO). Cette absence ou altération de la clairance mucociliaire peut être due à un dysfonctionnement du battement individuel des cils (mouvement ou motricité) et/ou de la coordination ciliaire.

L'observation *in vivo* des battements ciliaires afin d'évaluer la clairance mucociliaire est difficile. Dans le passé, l'évaluation *in vivo* de la clairance mucociliaire était réalisée grâce à des techniques utilisant la saccharine, une goutte de marqueur bleu ou la clairance de traceurs radioactifs. Une tomographie par cohérence micro-optique a également été proposée. Cependant, en raison des contraintes que ces différentes techniques imposent, notamment la bonne coopération du patient pour le test de saccharine, le caractère invasif pour l'examen endoscopique et/ou l'inhalation d'éléments radiopharmaceutiques, elles ont été quasiment abandonnées dans la pratique clinique actuelle. Actuellement, la méthode la plus couramment utilisée pour évaluer les battements ciliaires est l'étude *ex vivo* par microscopie en champ clair, souvent associée à l'analyse par vidéo-microscopie à haute vitesse (HSVM) de la fréquence de battement ciliaire des cellules ciliées, obtenues par brossage nasal ou bronchique. Une autre méthode employée pour évaluer la clairance mucociliaire consiste en le suivi du mouvement des microbilles de marquage du déplacement de fluide généré par le battement ciliaire par vélocimétrie par imagerie de particules (PIV). Cette méthode décrite dans l'article Bottier, M. et al. "A new index for characterizing micro-bead motion in a flow induced by ciliary beating: part II, modeling", PLoS Comput. Biol. 13(7): e1005552 (2017). Cette méthode s'appuie sur un modèle théorique du mouvement d'écoulement induit par le battement actif des cils, permettant d'évaluer la contrainte de cisaillement exercée par les cils sur le milieu. Or cette contrainte de cisaillement s'avère être un indice fiable pour caractériser l'efficacité du battement ciliaire. Cependant, cette méthode expérimentale d'évaluation du cisaillement présente une résolution spatiale faible et s'est avèrée longue et délicate à mettre en œuvre dans le cadre de la routine clinique.

Des travaux récents décrit dans l'article Kim, J., Michelin, S., Hilbers, M. et al. « Monitoring the orientation of rare-earth-doped nanorods for flow shear tomography. » Nature Nanotech 12, 914-919 (2017) permettent de cartographier le taux de cisaillement dans un microcanal dans lequel circule un fluide, via l'utilisation de nano-bâtonnets de phosphate de lanthane dopé à l'europium (LaPO₄:Eu) en suspension. Cette méthode n'a jamais été utilisée pour l'évaluation du comportement d'échantillons cellulaires dans un fluide et sa mise en œuvre nécessite de résoudre de nombreux problèmes, entre autres la stabilité des nano-bâtonnets dans le milieu adapté aux échantillons cellulaires, la non-toxicité cellulaire des nano-bâtonnets, et l'obtention d'une résolution spatiotemporelle permettant l'étude du comportement dynamique de l'échantillon cellulaire au niveau microscopique.

Il existe donc un besoin pour un procédé d'étude du comportement d'un échantillon cellulaire dans un fluide, rapide, présentant une bonne résolution, qui soit à la fois facile à mettre en œuvre en pratique clinique et peu coûteux.

### Exposé de l'invention

L'invention répond à ce besoin par un procédé d'étude du comportement d'un échantillon cellulaire contenu dans un milieu fluide contenant une pluralité de nanoparticules de forme anisotrope dispersées dans ce milieu , le procédé comportant :
(i) la détermination d'au moins une caractéristique d'orientation des nanoparticules dans une zone de mesure à l'interface entre le milieu fluide et l'échantillon cellulaire, l'orientation résultant au moins partiellement de l'interaction du milieu fluide et de l'échantillon cellulaire,
(ii) la détermination d'une caractéristique de cisaillement du milieu fluide dans la zone de mesure à partir de la caractéristique d'orientation des nanoparticules déterminée dans cette zone de mesure,
(iii) la détermination d'une caractéristique de l'échantillon cellulaire à partir de la caractéristique de cisaillement ainsi déterminé dans la zone de mesure.

Par *« caractéristique de cisaillement »,* on comprend la mesure d'une caractéristique du gradient de vitesse du fluide dans le milieu fluide à l'interface avec l'échantillon cellulaire, notamment sa direction et/ou sa valeur. Une telle mesure permet de caractériser la force de cisaillement exercée par l'échantillon cellulaire sur le milieu fluide.

Par *« nanoparticules de forme anisotrope* », on comprend des particules présentant un rapport d'aspect défini comme le rapport de la longueur sur la plus grande dimension transversale supérieur à 1, c'est-à-dire étant sensiblement allongée selon au moins une direction.

Par *« dispersées* », on comprend que les nanoparticules sont en suspension dans le milieu sans s'agréger entre elles.

Par *« l'orientation des nanoparticules* », on comprend que les nanoparticules, par leur forme anisotrope, peuvent s'orienter de façon statistique dans le milieu fluide en fonction des contraintes de cisaillement qu'ils subissent. Plus le cisaillement qu'ils subissent est élevé, plus les nanoparticules vont s'orienter dans une même direction correspondant sensiblement au sens d'écoulement du fluide et plus la distribution d'orientation sera étroite autour de cette direction.

Comme expliqué ci-dessus, les nanoparticules s'orientent dans le milieu fluide selon la force de cisaillement qu'elles subissent. La détermination d'au moins une caractéristique d'orientation des nanoparticules dans une zone de mesure permet de déterminer le taux de cisaillement dans la zone de mesure et d'en déduire une information quant à une caractéristique de l'échantillon cellulaire, notamment l'efficacité globale du battement ciliaire, par exemple par comparaison avec un taux de cisaillement local prédéterminée pour un échantillon cellulaire sain.

De préférence, le procédé est un procédé ex vivo ou en extemporané.

Le procédé est non thérapeutique en tant que tel.

### Echantillon cellulaire

De préférence, l'échantillon cellulaire est composé d'au moins un amas cellulaire notamment choisie parmi les cellules épithéliales ciliées battantes, en particulier un échantillon d'une bordure d'un épithélium cilié, les cellules flagellées, les cellules embryonnaires, les cellules sanguines, les cellules reproductrices, les globules rouges, les cellules du système immunitaire.

L'échantillon cellulaire peut être d'origine humaine, animale ou végétale.

### Milieu fluide

De préférence, le milieu fluide est liquide, notamment aqueux.

De préférence, le milieu fluide comporte une solution physiologiquement acceptable pour l'échantillon cellulaire, notamment un milieu Eagle modifié de Dulbecco (DMEM), pour maintenir la viabilité de l'échantillon cellulaire. Le cas échéant le milieu fluide peut contenir des nutriments pour l'échantillon cellulaire et constituer un milieu de culture.

De préférence, la circulation du fluide est induite par le mouvement à la surface de l'échantillon biologique sans action extérieur. Une telle circulation peut être, par exemple, la résultante de mouvements de cils à la surface de l'échantillon cellulaire.

### Nanoparticules

De préférence, les nanoparticules sont des nano-bâtonnets, c'est-à-dire des nanoparticules de forme générale allongée s'étendant principalement le long d'une ligne directrice, curviligne ou, de préférence, rectiligne. Dans ce cas, le rapport d'aspect est le rapport de la longueur, mesurée le long de cette ligne directrice, sur la largeur, la largeur étant la plus grande dimension qu'il est possible de mesurer dans l'ensemble des plans transversaux (perpendiculaires à la ligne directrice) le long de la ligne directrice.

De préférence, le rapport d'aspect des nanoparticules est supérieur ou égale à 3, mieux supérieure ou égale à 10.

De préférence, les nanoparticules sont configurées pour émettre un rayonnement de photoluminescence et/ou sont biréfringents.

De préférence, les nanoparticules présentent une émission de photoluminescence polarisée.

De préférence, les nanoparticules sont des nano-bâtonnets dopés par des terres rares. Les nanoparticules peuvent être d'oxyde ou de fluorure, notamment de phosphate de lanthane (LaPO₄), de fluorures de sodium et d'yttrium (NaYF4) ou leurs dérivés, dopé aux terres rares, notamment à l'europium. Les nano-bâtonnets peuvent avoir une symétrie cristalline, notamment une symétrie selon un axe parallèle à la ligne directrice du nano-bâtonnet.. Les nano-bâtonnets sont de préférence de LaPO₄ dopée à l'europium et peuvent être en phase cristalline rhabdophane, ou de préférence en phase cristalline monazite.

De tels nano-bâtonnets émettent chacun un rayonnement de photoluminescence qui est fortement polarisée en fonction de l'orientation des nano-bâtonnets. Il est alors possible, comme cela est décrit dans l'article Kim, J., Michelin, S., Hilbers, M. et al. « Monitoring the orientation of rare-earth-doped nanorods for flow shear tomography. » Nature Nanotech 12, 914-919 (2017), en mesurant à différents angles de polarisation le rayonnement de fluorescence polarisé à différents angles de polarisation des nano-bâtonnets dans une zone de mesure contenant plusieurs nano bâtonnets, notamment plusieurs centaines de nano-bâtonnets, de déterminer au moins une caractéristique d'orientation des nano-bâtonnets. Une différence de la forme du spectre aux différents angles de polarisation est caractéristique d'une orientation particulière des nanoparticules et donc de la présence d'une force de cisaillement dans la zone de mesure dans le milieu fluide.

De tels bâtonnets sont également biréfringents et la mesure d'au moins une caractéristique du profil de biréfringence dans une zone de mesure peut également permettre de déterminer une caractéristique d'orientation des nano-bâtonnets et d'en déduire une caractéristique de cisaillement dans la zone de mesure.

De préférence, les nanoparticules présentent une longueur moyenne inférieure ou égale à 1 µm, mieux inférieure ou égale à 200 nm. De préférence, l'écart type de la distribution de longueurs des nanoparticules est inférieur ou égal à 100 nm. Une telle distribution de nanoparticules permet d'avoir une bonne résolution spatiotemporelle de mesure et une bonne sensibilité de l'orientation des nanoparticules au cisaillement dans le milieu fluide.

De préférence, les nanoparticules comportent, en surface, des molécules liantes de stabilisation de la dispersion, notamment des molécules liantes présentant des groupes terminaux amine, silane (PEG-silane), ou d'acides alendroniques. De telles molécules améliorent la stabilité de la dispersion de nanoparticules dans le milieu fluide biologique en isolant 1 cœur des nanoparticules du milieu fluide. De plus, elles sont peu toxiques pour l'échantillon cellulaire.

De préférence, la concentration en nanoparticules dans le milieu fluide est inférieure ou égale à 10% en fraction volumique du milieu fluide.

### Photoluminescence

De préférence, les nanoparticules sont photoluminescentes et l'étape de détermination de la caractéristique d'orientation des nanoparticules comporte :
- l'excitation de photoluminescence des nanoparticules par une source lumineuse générant l'émission d'une lumière de photoluminescence par les nanoparticules, et
- la mesure dans la zone de mesure d'au moins deux informations spectrales de la lumière de photoluminescence polarisée à une ou plusieurs polarisations différentes,
- la détermination de la caractéristique d'orientation des nanoparticules à partir des mesures des au moins deux informations spectrales de la lumière de photoluminescence polarisée à une ou plusieurs polarisations différentes.

De préférence, la mesure des deux informations spectrales comporte la détection optique de la lumière de photoluminescence des nanoparticules à l'aide d'un système de microscopie optique, notamment confocal. Le système de microscopie peut comporter un microscope optique confocal, et un ou plusieurs capteurs optiques de la lumière émise par photoluminescence par les nanoparticules. Le ou les capteurs optiques peuvent être des photodiodes, notamment des tubes photomultiplicateurs (PMT), des photodiodes à avalanches (APD), combinés avec des filtres optiques spectrales ou spectromètre résolu en longueur d'onde.

Le procédé peut comporter la rotation d'un filtre polarisant pour changer l'angle de polarisation, notamment agencé entre le milieu fluide et le système optique, notamment le ou les capteurs optiques pour permettre les mesures des au moins deux informations spectrales de la lumière de photoluminescence polarisée à une ou plusieurs polarisations différentes.

De préférence, la source lumineuse d'excitation est une source laser configurée pour exciter les nanoparticules à une longueur d'onde optimale prédéterminée correspondante notamment à un pic d'excitation, notamment comprise entre 250 nm et 550 nm, notamment pour le LaPO₄:Eu à une longueur d'onde de 394 nm.

En variante, la source lumineuse d'excitation émet une lumière ayant un spectre de longueur d'onde large, notamment une lumière blanche.

Dans le cas du LaPO₄:Eu, les au moins deux informations spectrales mesurées peuvent correspondre aux spectres entre 570 et 720 nm dans laquelle sont situées les bandes de transitions de l'ion Eu³⁺

Le procédé peut comporter la mesure de l'intensité de la lumière polarisée à un ou plusieurs angles de polarisation différents à au moins deux longueurs d'onde données différentes, les longueurs d'onde données correspondant dans le spectre de la lumière émise par les nanoparticules à deux pics d'intensité distinct du spectre de la lumière de photoluminescence, notamment pour des nano-bâtonnets de LaPO₄:Eu, étant compris
- entre 580 nm et 590 nm, et entre 590 et 600 nm respectivement,
- entre 610 nm et 617 nm et entre 617 et 630 nm respectivement, ou
- entre 680 nm et 690 nm et entre 690 et 710 nm respectivement.

De préférence, l'angle entre les deux angles de polarisation est supérieur ou égal à 10°, mieux supérieur ou égale à 30°, encore mieux supérieur ou égal à 60°, par exemple égal à 90°.

Le procédé peut comporter l'identification d'une différence d'intensité de la lumière polarisée aux deux angles de polarisation différents à une longueur d'onde donnée correspondant à une orientation préférentielle des nanoparticules dans une direction particulière.

Le procédé peut comporter la mesure d'une pluralité d'informations spectrales de la lumière de photoluminescence polarisée, notamment l'intensité de la lumière polarisée à une gamme de longueurs d'onde donnée ou le profil spectral de la lumière polarisée, à plusieurs angles de polarisation différents.

En variante, le procédé comporte
- la mesure du profil de biréfringence de la distribution de nanoparticules dans une zone de mesure,
- la détermination d'une caractéristique d'orientation des nanoparticules à
partir de la mesure de biréfringence de la distribution de nanoparticules dans une zone de mesure.

De préférence, la mesure des informations spectrales de la lumière de photoluminescence polarisée à une ou plusieurs polarisations différentes se fait temporellement après l'excitation de photoluminescence des nanoparticules par une source lumineuse, notamment au moins 5 microsecondes, mieux au moins 10 microsecondes après pour éliminer la fluorescence parasite.

### Orientation

De préférence, la caractéristique de cisaillement déterminée dans la zone de mesure est la valeur de cisaillement moyenne dans la zone de mesure.

De préférence, le procédé comporte la détermination d'au moins deux caractéristiques de l'orientation des nanoparticules, l'orientation des nanoparticules et le paramètre d'ordre associé caractéristique de la dispersion de l'orientation des nanoparticules par rapport à l'orientation dans la zone de mesure. Pour cela la méthode de détermination est telle que décrite dans l'article Kim, J., Michelin, S., Hilbers, M. et al. « Monitoring the orientation of rare-earth-doped nanorods for flow shear tomography. » Nature Nanotech 12, 914-919 (2017), ici incorporé en référence.

De préférence, le procédé comporte la détermination de la direction de cisaillement et de la valeur de cisaillement dans la zone de mesure à partir des deux caractéristiques d'orientation des nanoparticules déterminée dans cette zone de mesure.

### Zone de mesure

De préférence, la zone de mesure correspond au volume focal du système de microscopie optique. Il peut présenter un volume inférieur ou égale à 3 µm³, mieux 1 µm³.

### Mode dynamique

Le procédé peut comporter la détermination de la caractéristique de cisaillement dynamique du milieu fluide dans la zone de mesure par la méthode décrite précédemment à partir de mesures continues dans le temps dans la même zone de mesure et la détermination d'une caractéristique de l'échantillon cellulaire à partir de la caractéristique de cisaillement dynamique ainsi déterminé dans la zone de mesure.

### Mode de scan

Le procédé peut comporter :
- le balayage du milieu fluide selon au moins deux directions par le système de mesure,
- la détermination d'au moins une caractéristique d'orientation des nanoparticules à chaque position du système de mesure,
- la détermination d'une cartographie de la caractéristique de cisaillement dans le milieu fluide à partir d'au moins une caractéristique d'orientation des nanoparticules mesurée en chaque position du système de mesure.

### Caractérisation de l'échantillon cellulaire

De préférence, le procédé comporte la comparaison de la caractéristique de cisaillement dans la zone de mesure déterminée ou de la cartographie de la caractéristique de cisaillement à une caractéristique de cisaillement de référence ou une cartographie de référence de la caractéristique de cisaillement préétablie, notamment pour un échantillon cellulaire de référence.

Le procédé peut comporter la caractérisation, notamment l'efficience de l'échantillon cellulaire à partir de la caractéristique de cisaillement dans la zone de mesure déterminée ou de la cartographie de la caractéristique de cisaillement déterminée.

De préférence, l'échantillon cellulaire est de cellules épithéliales ciliées et le procédé comporte la détermination de l'efficacité du battement ciliaire desdites cellules par comparaison du taux de cisaillement déterminée ou de la cartographie du taux de cisaillement déterminée et d'un taux de cisaillement ou d'une cartographie de référence préétablie correspondant à un battement normal des cils des cellules épithéliales ciliées.

De préférence, l'échantillon cellulaire est obtenu par brossage nasal.

De préférence, le temps entre l'obtention de l'échantillon cellulaire et les mesures est inférieur ou égal à 3h.

### Dispositif

L'invention a également pour objet un dispositif d'étude d'un échantillon cellulaire, notamment pour la mise en œuvre du procédé défini plus haut, comprenant les caractéristiques de la revendication 14 ci-jointe.

Le dispositif comporte un processeur configuré pour déterminer au moins une caractéristique d'orientation des nanoparticules dans la zone de mesure.

De préférence, la chambre est une chambre microfluidique.

Le dispositif peut comporter l'échantillon cellulaire dans le milieu fluide.

Les propriétés du procédé décrits ci-dessus s'appliquent au dispositif, seules ou en combinaison.

Le dispositif comporté un processeur configuré pour déterminer la caractéristique de cisaillement dans la zone de mesure à partir de l'information caractéristique de l'orientation des nanoparticules déterminée par le système de mesure dans cette zone de mesure, notamment à partir de la caractéristique d'orientation des nanoparticules déterminée.

De préférence, le système de mesure comporte un système de balayage selon au moins deux directions, mieux trois directions, pour déplacer le système de mesure entre plusieurs mesures de sorte à prendre des mesures dans plusieurs zones de mesure différentes. Le processeur peut déterminer une cartographie du taux de cisaillement dans le milieu fluide en fonction des informations d'orientation afin d'établir une cartographie du taux de cisaillement dans le milieu fluide à partir des informations d'orientation des nanoparticules mesurées par le système de mesure dans différentes zones de mesures.

De préférence, le système de mesure comporte :
- une source lumineuse d'excitation photoluminescente des nanoparticules,
- un système de microscopie optique, notamment comportant :
   ∘ un microscope optique confocal, et
   ∘ un ou des capteurs optiques de la lumière émise par photoluminescence par les nanoparticules,
- un filtre polarisant mobile en rotation disposé entre la chambre et le système de microscopie optique, notamment le ou les capteurs optiques.

Le ou les capteurs optiques peuvent être configuré(s) pour prendre une mesure retardée par rapport à la source lumineuse d'excitation photoluminescente.

### Brève description des dessins

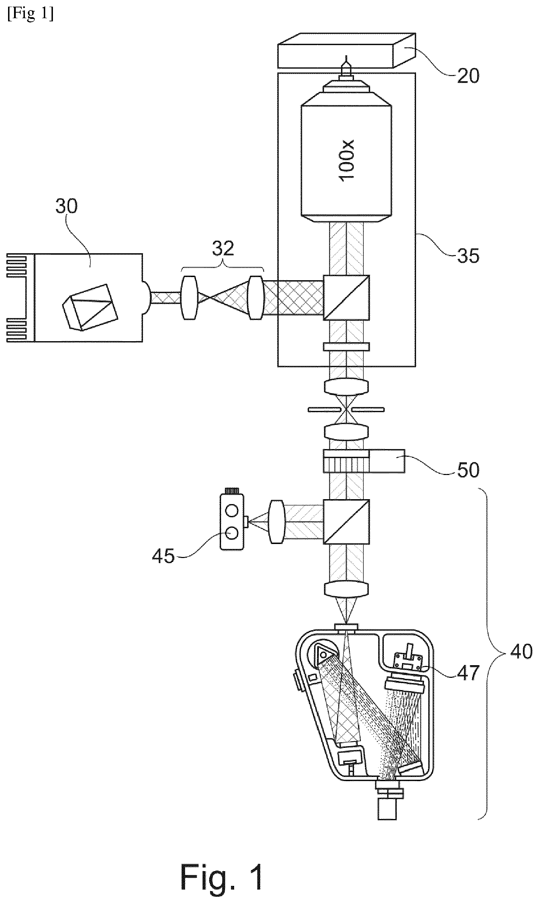

[Fig 1] La figure 1 représente schématiquement un exemple de chambre microfluidique,
[Fig 2] la figure 2 représente schématiquement un exemple de dispositif, et
[Fig 3] la figure 3 représente schématiquement le battement de cils de cellules épithéliales ciliées en trois dimensions.

### Description détaillée

Un exemple de procédé d'étude d'un amas de cellules épithéliales ciliées selon l'invention est décrit ci-dessous.

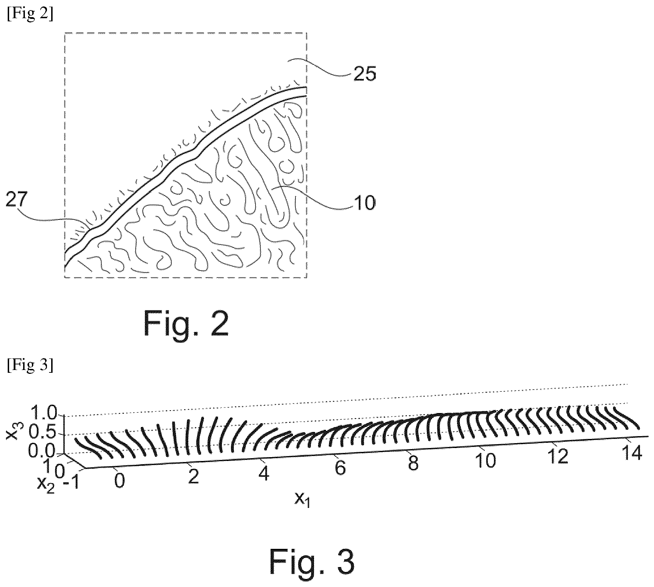

Tout d'abord, des cellules épithéliales ciliées 10 sont mises en culture dans une chambre 20 contenant une solution physiologique 25 contenant des nano-bâtonnets. Les cellules épithéliales ciliées présentent en surface des cils 27 qui présentent, dans le cas de cellules saines, un mouvement permanent, périodique et synchronisé avec celui des cils avoisinants et ceux des cellules voisines avec un décalage de phase qui s'accroît avec la distance. Le mouvement des cils suit le mouvement d'une onde métachrone, telle que représentée en trois dimensions à la figure 3. La chambre de culture peut être une plaque de microscope, une boite de pétri ou préférentiellement une chambre microfluidique.

Les nano-bâtonnets sont en phosphate de lanthane dopée à l'europium (LaPO4:Eu) de phase cristalline rhabdophane ou monazite, de préférence monazite et comportent à leur surface des molécules liantes de polyéthylène glycol-silane (PEG-silane). Les nano-bâtonnets dans la solution physiologique présentent une distribution de longueur caractérisée par une longueur moyenne comprise entre 100 nm et 500 nm avec un écart type inférieur ou égal à 200 nm et une distribution de plus grande dimension de la section transversale caractérisée par une plus grande dimension moyenne comprise entre 20 nm et 5 nm avec un écart type inférieur ou égal à 10 nm.

Une zone de mesure de la chambre 20 est ensuite éclairée par une source lumineuse, notamment un laser sensiblement monochromatique 30 à une longueur d'onde de 395 nm dont la lumière émise est transmise de façon conventionnelle vers l'entrée lumineuse d'un microscope optique 35 de sorte à éclairer la chambre dans la zone de mesure. La source lumineuse 30 permet d'exciter la photoluminescence des nano-bâtonnets à une longueur d'onde de 395 nm correspondant au pic d'excitation de la transition ⁷F₀-⁵L₆ pour Eu³⁺. Les spectres entre 580 nm et 720 nm de la lumière de photoluminescence émise par les nano-bâtonnets excités dans la zone de mesure sont alors mesurés à deux angles de polarisation différents, ici orthogonaux l'un par rapport à l'autre, à l'aide d'un système de mesure 40 et d'un polariseur 50 mobile en rotation disposé entre la chambre microfluidique 20 et le système de mesure 40. La lumière émise par les nano-bâtonnets est transmise au système de mesure 40 par l'intermédiaire du microscope optique 35, le polariseur 50 étant disposé entre le microscope optique 35 et le système de mesure 40. Le microscope optique peut comporter un système de balayage, notamment un dispositif de balayage piézoélectrique.

La zone de mesure présente une plus grande dimension inférieure ou égale à 2 µm, le système de mesure présentant une résolution inférieure ou égale à 1 µm³.

Le balayage de la chambre microfluidique selon deux directions à l'aide du système de balayage, permet la mesure en à chaque point de la chambre de mesure.

Les spectres de photoluminescence émis par les nano-bâtonnets dans chaque zone de mesure entre 580 et 720 nm aux deux angles de polarisation permettent alors de déterminer un paramètre d'ordre f, compris entre 0 et 1, quantifiant la dispersion locale de l'orientation des nano-bâtonnets, 0 correspondant à une orientation désordonnée des nano-bâtonnets et 1 à des nano-bâtonnets totalement alignés, et une directrice *̅n̅*̅ correspondant à l'orientation moyenne des nano-bâtonnets comme cela est décrit en pages 6 et 7 de l'annexe de l'article Kim, J., Michelin, S., Hilbers, M. et al. Supplementary information to « Monitoring the orientation of rare-earth-doped nanorods for flow shear tomography ». Nature Nanotech 12, 914-919 (2017).

La valeur du taux de cisaillement dans la zone de mesure en chaque point de la chambre de mesure est alors déterminée à partir du paramètre d'ordre f et l'orientation du cisaillement est donnée par l'orientation moyenne des nano-bâtonnets *̅n̅*̅, comme cela est décrit en pages 7 à 11 de l'annexe de l'article Kim, J., Michelin, S., Hilbers, M. et al. Supplementary information to « Monitoring the orientation of rare-earth-doped nanorods for flow shear tomography ». Nature Nanotech 12, 914-919 (2017).

Une cartographie du cisaillement à l'interface entre le milieu fluide et la ou les cellules ciliées peut alors être établie. Une telle cartographie est comparée à la cartographie de cellules ciliées saines et on peut alors déterminer si les cellules ciliées étudiées ont un fonctionnement proche ou éloigné des cellules saines de référence. En effet, à l'état physiologique, les cils situés à la partie apicale des cellules épithéliales ciliées battent de façon synchrone et génère une force de cisaillement sur le fluide, comme cela est décrit dans l'article Bottier, M. et al. "A new index for characterizing micro-bead motion in a flow induced by ciliary beating: part II, modeling", PLoS Comput. Biol. 13(7): e1005552 (2017). Ainsi, lorsque les cils battent de manière asynchrone ou lorsque le battement est absent, le cisaillement détecté par la méthode décrite ci-dessus est faible voire inexistant. Les informations déterminées par la méthode décrite permettent donc de caractériser l'efficacité du battement des cellules ciliées étudiées.

En variante, les spectres de photoluminescence émis par les nano-bâtonnets dans une zone de mesure entre 580 et 720 nm aux deux angles de polarisation est mesurée en continue dans le temps avec des acquisitions espacées d'un temps d'environ 1µs. La valeur du taux de cisaillement et l'orientation du cisaillement sont alors déterminées pour chacune des mesures comme décrit précédemment pour en déduire le comportement dynamique de l'échantillon cellulaire 10, notamment le mouvement des cils 27 de ce dernier, dans la zone de mesure.

L'invention n'est pas limitée à l'exemple qui vient d'être décrit. Par exemple, la matière biologique ne se limite pas aux cellules ciliées épithéliales des voies aériennes mais peut aussi être appliqué à la mesure des cellules ciliées de l'endosalpinx des trompes de Fallope, aux cellules flagellés, tels que le sperme ou les embryons, ou encore à des organismes tels les paramécies (protozoaire cilié unicellulaire) utilisées comme modèle pour l'étude des cils.

En variante, il est aussi possible de déterminer une caractéristique d'orientation locale des nano-bâtonnets à partir de la mesure de la biréfringence des nano-bâtonnets dans le milieu fluide à l'interface avec la matière biologique, pour en déduire le taux de cisaillement local.

En variante, les nanoparticules sont des nanoparticules de forme anisotrope autres que celles mentionnées de nano-bâtonnet, l'important étant qu'une signature physique de l'orientation de ces derniers existe et soit suffisamment forte et caractéristique pour permettre l'identification de l'orientation générales des nanoparticules et d'en déduire le taux de cisaillement.

En variante, les nanoparticules comportent en surface des molécules liantes autres permettant à la fois la bonne dispersion des nanoparticules dans le milieu fluide et ayant une faible toxicité pour la matière biologique ou sont dépourvues de molécules liantes en surface, en particulier quand les nanoparticules sont en suspension suffisamment stable.

## Revendications

1. Procédé d'étude du comportement d'une matière biologique (10) contenue dans un milieu fluide (25) contenant une pluralité de nanoparticules de forme anisotrope dispersées dans ce milieu, le procédé comportant :
(i) la détermination d'au moins une caractéristique d'orientation des nanoparticules dans une zone de mesure à l'interface entre le milieu fluide (25),et la matière biologique (10), l'orientation résultant au moins partiellement de l'interaction du milieu fluide (25) et de la matière biologique (10),
(ii) la détermination d'une caractéristique de cisaillement moyen du milieu fluide dans la zone de mesure à partir de ladite au moins une caractéristique d'orientation nanoparticules déterminée dans cette zone de mesure,
(iii) la détermination d'une caractéristique de l'échantillon cellulaire (10) à partir du taux de cisaillement moyen ainsi déterminé dans la zone de mesure.

2. Procédé selon la revendication 1, dans lequel l'échantillon cellulaire est composé d'au moins un amas cellulaire notamment choisie parmi les cellules épithéliales ciliées battantes, en particulier un échantillon d'une bordure d'un épithélium cilié, les cellules flagellées, les cellules embryonnaires, les cellules sanguines, les cellules reproductrices, les globules rouges, les cellules du système immunitaire.

3. Procédé selon l'une quelconque des revendications précédentes, dans lequel les nanoparticules présentent une émission de photoluminescence polarisée.

4. Procédé selon l'une quelconque des revendications précédentes, dans lequel les nanoparticules sont des nano-bâtonnets dopés par des terres rares, notamment des nano-bâtonnets d'oxyde ou de fluorure, notamment de phosphate de lanthane (LaPO₄), de fluorures de sodium et d'yttrium (NaYF₄) ou leurs dérivés, dopé aux terres rares, notamment à l'europium, les nano-bâtonnets étant notamment de LaPO₄ dopée à l'europium en phase cristalline rhabdophane, ou de préférence en phase cristalline monazite.

5. Procédé selon l'une quelconque des revendications précédentes, dans lequel les nanoparticules présentent une longueur moyenne inférieure ou égale à 1 µm, mieux inférieure ou égale à 200 nm, et un écart type de la distribution de longueurs des nanoparticules inférieur ou égal à 100 nm.

6. Procédé selon l'une quelconque des revendications précédentes, dans lequel le rapport d'aspect les nanoparticules présentent un rapport d'aspect supérieur ou égal à 3, mieux supérieure ou égale à 10.

7. Procédé selon l'une quelconque des revendications précédentes, dans lequel la concentration en nanoparticules dans le milieu fluide (25) est inférieure ou égale à 10% en fraction volumique du milieu fluide (25).

8. Procédé selon l'une quelconque des revendications précédentes, comportant la détermination d'au moins deux caractéristiques de l'orientation des nanoparticules, l'orientation des nanoparticules et le paramètre d'ordre associé caractéristique de la dispersion de l'orientation des nanoparticules par rapport à l'orientation dans la zone de mesure, le procédé comportant de préférence la détermination de la direction de cisaillement et de la valeur de cisaillement dans la zone de mesure à partir des deux caractéristiques d'orientation des nanoparticules déterminée dans cette zone de mesure.

9. Procédé selon l'une quelconque des revendications précédentes, dans lequel les nanoparticules sont photoluminescentes et l'étape de détermination de la caractéristique d'orientation des nanoparticules comporte :
- l'excitation de photoluminescence des nanoparticules par une source lumineuse (30) générant l'émission d'une lumière de photoluminescence par les nanoparticules, et
- la mesure dans la zone de mesure d'au moins deux informations spectrales de la lumière de photoluminescence polarisée à une ou plusieurs polarisations différentes,
- la détermination de la caractéristique d'orientation des nanoparticules à partir des mesures des au moins deux informations spectrales de la lumière de photoluminescence polarisée à une ou plusieurs polarisations différentes.

10. Procédé selon la revendication 9, dans lequel les nanoparticules sont des nano-bâtonnets de LaPO₄ dopée à l'europium, au moins deux informations spectrales mesurées correspondant aux spectres entre 570 et 720 nm dans laquelle sont situées les bandes de transitions de l'ion Eu3+.

11. Procédé selon la revendication 9 ou 10, comportant la mesure de l'intensité de la lumière polarisée à un ou plusieurs angles de polarisation différents à au moins deux longueurs d'onde données différentes, les longueurs d'onde données correspondant dans le spectre de la lumière émise par les nanoparticules à deux pics d'intensité distinct du spectre de la lumière de photoluminescence, notamment pour des nano-bâtonnets de LaPO₄ dopée à l'europium étant compris entre 580 nm et 590 nm, et entre 590 et 600 nm respectivement, entre 610 nm et 617 nm et entre 617 et 630 nm respectivement, ou entre 680 nm et 690 nm et entre 690 et 710 nm respectivement.

12. Procédé selon l'une quelconque des revendications précédentes, comportant :
- le balayage du milieu fluide (25) selon au moins deux directions, mieux trois directions, par le système de mesure,
- la détermination d'au moins une caractéristique d'orientation des nanoparticules à chaque position du système de mesure,
- la détermination d'une cartographie du taux de cisaillement dans le milieu fluide (25) à partir de ladite au moins une caractéristique d'orientation des nanoparticules mesurée en chaque position du système de mesure.

13. Procédé selon l'une quelconque des revendications précédentes, comportant la comparaison de la caractéristique de cisaillement dans la zone de mesure déterminée ou de la cartographie de la caractéristique de cisaillement à une caractéristique de cisaillement de référence ou une cartographie de référence de la caractéristique de cisaillement préétablie, notamment pour un échantillon cellulaire de référence.

14. Dispositif d'étude d'un échantillon cellulaire, notamment pour la mise en œuvre du procédé défini plus haut, comportant :
- une chambre fluidique (20) configurée pour recevoir les composants suivants :
o un milieu fluide (25),
o l'échantillon cellulaire (10) dans le milieu fluidique (25), et
o une pluralité de nanoparticules dispersés dans le milieu fluide (25),
- un système de mesure d'au moins une information caractéristique de l'orientation des nanoparticules dans une zone de mesure à l'interface entre le milieu fluide (25) et l'échantillon cellulaire ;
le dispositif comportant en outre un processeur configuré pour déterminer une caractéristique de cisaillement dans la zone de mesure à partir de l'information caractéristique de l'orientation des nanoparticules déterminée par le système de mesure dans cette zone de mesure, notamment à partir de la caractéristique d'orientation des nanoparticules déterminée.

## Patentansprüche

1. Verfahren zur Untersuchung des Verhaltens eines biologischen Materials (10), das in einem fluiden Medium (25) enthalten ist, das eine Vielzahl von Nanopartikeln von anisotroper Form enthält, die in diesem Medium dispergiert sind, das Verfahren umfassend:
(i) das Bestimmen mindestens einer Ausrichtungseigenschaft der Nanopartikel in einer Messzone an der Grenzfläche zwischen dem fluiden Medium (25) und dem biologischen Material (10), wobei die Ausrichtung mindestens teilweise aus der Wechselwirkung des fluiden Mediums (25) und des biologischen Materials (10) resultiert,
(ii) das Bestimmen einer Eigenschaft der mittleren Scherung des fluiden Mediums in der Messzone ausgehend von der in dieser Messzone bestimmten mindestens einen Nanopartikel-Ausrichtungseigenschaft,
(iii) das Bestimmen einer Eigenschaft der Zellprobe (10) ausgehend von der in der Messzone so bestimmten mittleren Scherrate.

2. Verfahren nach Anspruch 1, wobei die Zellprobe aus mindestens einem Zellcluster besteht, insbesondere ausgewählt aus schlagenden Flimmerepithelzellen, insbesondere einer Probe eines Saums eines Flimmerepithels, flagellierten Zellen, embryonalen Zellen, Blutzellen, Reproduktionszellen, roten Blutkörperchen, Zellen des Immunsystems.

3. Verfahren nach einem der vorhergehenden Ansprüche, wobei die Nanopartikel eine polarisierte Photolumineszenzemission aufweisen.

4. Verfahren nach einem der vorhergehenden Ansprüche, wobei die Nanopartikel mit seltenen Erden dotierte Nanostäbchen sind, insbesondere Oxid- oder Fluorid-Nanostäbchen, insbesondere von Lanthanphosphat (LaPO₄), Natrium-Yttrium-Fluoriden (NaYF₄) oder ihren Derivaten, dotiert mit seltenen Erden, insbesondere mit Europium, wobei die Nanostäbchen insbesondere aus mit Europium dotiertem LaPO₄ in kristalliner Rhabdophanphase oder bevorzugt in kristalliner Monazitphase sind

5. Verfahren nach einem der vorhergehenden Ansprüche, wobei die Nanopartikel eine durchschnittliche Länge von 1 µm oder weniger, besser von 200 nm oder weniger, und eine Standardabweichung der Längenverteilung der Nanopartikel von 100 nm oder weniger aufweisen.

6. Verfahren nach einem der vorhergehenden Ansprüche, wobei das Aspektverhältnis die Nanopartikel ein Aspektverhältnis größer oder gleich 3, bevorzugt größer oder gleich 10 aufweisen.

7. Verfahren nach einem der vorhergehenden Ansprüche, wobei die Konzentration an Nanopartikeln in dem fluiden Medium (25) 10 % oder weniger an Volumenanteil des fluiden Mediums (25) beträgt.

8. Verfahren nach einem der vorhergehenden Ansprüche, umfassend das Bestimmen von mindestens zwei Eigenschaften der Ausrichtung der Nanopartikel, die Ausrichtung der Nanopartikel und den zugeordneten Ordnungsparameter, der charakteristisch für die Dispersion der Ausrichtung der Nanopartikel im Verhältnis zu der Ausrichtung in der Messzone ist, wobei das Verfahren bevorzugt das Bestimmen der Scherrichtung und des Scherwerts in der Messzone ausgehend von den beiden in dieser Messzone bestimmten Ausrichtungseigenschaften der Nanopartikel umfasst.

9. Verfahren nach einem der vorhergehenden Ansprüche, wobei die Nanopartikel photolumineszent sind und der Schritt des Bestimmens der Ausrichtungseigenschaft der Nanopartikel umfasst:
- die Photolumineszenzanregung der Nanopartikel durch eine Lichtquelle (30), die die Emission eines Photolumineszenzlichts durch die Nanopartikel erzeugt, und
- das Messen in der Messzone von mindestens zwei spektralen Informationen des auf eine oder mehrere unterschiedliche Polarisationen polarisierten Photolumineszenzlichts,
- das Bestimmen der Ausrichtungseigenschaft der Nanopartikel ausgehend von den Messungen der mindestens zwei spektralen Informationen des auf eine oder mehrere unterschiedliche Polarisationen polarisierten Photolumineszenzlichts.

10. Verfahren nach Anspruch 9, wobei die Nanopartikel Nanostäbchen von mit Europium dotiertem LaPO₄ sind, wobei mindestens zwei gemessene spektrale Informationen Spektren zwischen 570 und 720 nm entsprechen, in denen die Übergangsbanden des Ions Eu3+ liegen.

11. Verfahren nach Anspruch 9 oder 10, umfassend das Messen der Intensität des auf einen oder mehrere unterschiedliche Polarisationswinkel polarisierten Lichts mit mindestens zwei unterschiedlichen gegebenen Wellenlängen, wobei die gegebenen Wellenlängen, die in dem Spektrum des von den Nanopartikeln emittierten Lichts zwei verschiedenen Intensitätspeaks des Spektrums des Photolumineszenzlichts entsprechen, insbesondere für mit Europium dotierte Nanostäbchen von LaPO₄, zwischen 580 nm und 590 nm bzw. zwischen 590 und 600 nm, zwischen 610 nm und 617 nm bzw. zwischen 617 und 630 nm und zwischen 680 und 690 nm bzw. zwischen 690 und 710 nm liegen.

12. Verfahren nach einem der vorhergehenden Ansprüche, umfassend
- das Abtasten des fluiden Mediums (25) in mindestens zwei Richtungen, besser drei Richtungen, durch das Messsystem,
- das Bestimmen mindestens einer Ausrichtungseigenschaft der Nanopartikel für jede Position des Messsystems,
- das Bestimmen einer Kartographie der Scherrate in dem fluiden Medium (25) ausgehend von der an jeder Position des Messsystems gemessenen mindestens einen Ausrichtungseigenschaft der Nanopartikel.

13. Verfahren nach einem der vorhergehenden Ansprüche, umfassend das Vergleichen der Schereigenschaft in der bestimmten Messzone oder der Kartographie der Schereigenschaft mit einer Referenzschereigenschaft oder einer Referenzkartographie der vorbestimmten Schereigenschaft, insbesondere für eine Referenzzellprobe.

14. Vorrichtung zur Untersuchung einer Zellprobe, insbesondere zur Durchführung des weiter oben definierten Verfahrens, umfassend:
- eine fluidische Kammer (20), die dazu ausgelegt ist, die folgenden Komponenten aufzunehmen:
o ein fluides Medium (25),
o die Zellprobe (10) in dem fluidischen Medium (25), und
o eine Vielzahl von Nanopartikeln, die in dem fluiden Medium dispergiert sind (25),
- ein System zum Messen mindestens einer Information, die für die Ausrichtung der Nanopartikel in einer Messzone an der Grenzfläche zwischen dem fluiden Medium (25) und der Zellprobe charakteristisch ist;
wobei die Vorrichtung ferner einen Prozessor umfasst, der dazu ausgelegt ist, eine Schereigenschaft in der Messzone ausgehend von der für die Ausrichtung der Nanopartikel charakteristischen Information, die von dem Messsystem in der Messzone bestimmt wird, insbesondere ausgehend von der bestimmten Ausrichtungseigenschaft der Nanopartikel, zu bestimmen.

## Claims

1. Process for studying the behaviour of a biological material (10) contained in a fluid medium (25) containing a plurality of anisotropically-shaped nanoparticles dispersed in this medium, the process involving:
(i) determining at least one orientation characteristic of the nanoparticles in a measurement zone at the interface between the fluid medium (25) and the biological material (10), the orientation resulting at least partly from the interaction of the fluid medium (25) and the biological material (10),
(ii) determining a mean shear characteristic of the fluid medium in the measurement zone from said at least one nanoparticle orientation characteristic determined in this measurement zone,
(iii) determining a characteristic of the cell sample (10) from the mean shear rate thus determined in the measurement zone.

2. Process according to Claim 1, in which the cell sample is composed of at least one cell cluster notably chosen from beating ciliated epithelial cells, in particular a sample of a border of a ciliated epithelium, flagellated cells, embryonic cells, blood cells, reproductive cells, red blood cells and immune system cells.

3. Process according to either one of the preceding claims, in which the nanoparticles exhibit polarized photoluminescence emission.

4. Process according to any one of the preceding claims, in which the nanoparticles are rare-earth-doped nanorods, notably nanorods of oxide or fluoride, notably of lanthanum phosphate (LaPO₄), sodium yttrium fluoride (NaYF₄) or derivatives thereof, doped with rare-earth elements, notably with europium, the nanorods notably being europium-doped LaPO₄ in rhabdophane crystal phase, or preferably in monazite crystal phase.

5. Process according to any one of the preceding claims, in which the nanoparticles have a mean length of less than or equal to 1 µm, better still less than or equal to 200 nm, and a standard deviation of the nanoparticle length distribution of less than or equal to 100 nm.

6. Process according to any one of the preceding claims, in which the aspect ratio the nanoparticles have an aspect ratio of greater than or equal to 3, better still greater than or equal to 10.

7. Process according to any one of the preceding claims, in which the nanoparticle concentration in the fluid medium (25) is less than or equal to 10% as a volumetric fraction of the fluid medium (25).

8. Process according to any one of the preceding claims, which involves determining at least two characteristics of the nanoparticle orientation, the nanoparticle orientation and the associated order parameter characteristic of the dispersion of the nanoparticle orientation relative to the orientation in the measurement zone, the process preferably involving determining the shear direction and shear value in the measurement zone from the two nanoparticle orientation characteristics determined in this measurement zone.

9. Process according to any one of the preceding claims, in which the nanoparticles are photoluminescent and the step for determining the orientation characteristic of the nanoparticles involves:
- photoluminescence excitation of the nanoparticles by a light source (30), causing the nanoparticles to emit photoluminescent light, and
- measuring at least two items of spectral information of the polarized photoluminescence light at one or more different polarizations in the measurement zone,
- determining the orientation characteristic of the nanoparticles from the measurements of the at least two items of spectral information of the polarized photoluminescence light at one or more different polarizations.

10. Process according to Claim 9, in which the nanoparticles are europium-doped LaPO₄ nanorods, at least two measured items of spectral information corresponding to the spectra between 570 and 720 nm in which the transition bands of the Eu³⁺ ion are located.

11. Process according to Claim 9 or 10, which involves measuring the intensity of light polarized at one or more different polarization angles at at least two different given wavelengths, the given wavelengths corresponding in the spectrum of light emitted by the nanoparticles to two different intensity peaks in the spectrum of the photoluminescence light, notably for europium-doped LaPO₄ nanorods being between 580 nm and 590 nm, and between 590 nm and 600 nm, respectively, between 610 nm and 617 nm and between 617 nm and 630 nm, respectively, or between 680 nm and 690 nm and between 690 nm and 710 nm, respectively.

12. Process according to any one of the preceding claims, comprising:
- scanning the fluid medium (25) in at least two directions, better still three directions, by the measuring system,
- determining at least one nanoparticle orientation characteristic at each position of the measuring system,
- mapping the shear rate in the fluid medium (25) from said at least one nanoparticle orientation characteristic measured at each position of the measuring system.

13. Process according to any one of the preceding claims, which involves comparing the shear characteristic in the determined measurement zone or the mapping of the shear characteristic to a reference shear characteristic or a reference mapping of the pre-established shear characteristic, notably for a reference cell sample.

14. Device for studying a cell sample, notably for performing the process defined above, including:
- a fluidic chamber (20) configured to receive the following components:
∘ a fluid medium (25),
∘ the cell sample (10) in the fluid medium (25), and
∘ a plurality of nanoparticles dispersed in the fluid medium (25),
- a system for measuring at least one item of information characteristic of the orientation of the nanoparticles in a measurement zone at the interface between the fluid medium (25) and the cell sample;
the device further including a processor configured to determine a shear characteristic in the measurement zone from information characteristic of the nanoparticle orientation determined by the measurement system in this measurement zone, notably from the determined nanoparticle orientation characteristic.
